# EUROPEAN PATENT SPECIFICATION

(11) **EP 2 349 035 B1**
(45) Date of publication and mention of the grant of the patent: **22.10.2014**
(21) Application number: 09803241.0
(22) Date of filing: 23.07.2009
(51) Int. Cl.: A61B 17/58, A61B 17/70

(54) **DYNAMIC SPINAL STABILIZATION ASSEMBLY WITH TORSION AND SHEAR CONTROL**
DYNAMISCHE WIRBELSÄULENSTABILISATIONSANORDNUNG MIT TORSIONS- UND SCHERKONTROLLE
ENSEMBLE STABILISATION VERTÉBRALE DYNAMIQUE AVEC RÉGLAGE DE LA TORSION ET DU CISAILLEMENT

(30) Priority: 01.08.2008 US 221442
(43) Date of publication of application: 03.08.2011
(73) Proprietor: Jackson, Roger P., North Kansas City, MO 64116-3250 (US)
(72) Inventor: Jackson, Roger P., North Kansas City, MO 64116-3250 (US)
(74) Representative: Powell, Stephen David
(86) International application number: PCT/US2009/004270
(87) International publication number: WO 2010/014174

(56) References cited:
- WO-A2-2005/013839
- WO-A2-2008/013892
- WO-A2-2008/045210
- US-A1- 2008 140 076
- US-A1- 2008 177 317

## Description

### Background of the Invention

The present invention relates to apparatuses for use in performing spinal surgery and, in particular, to bone attachment structures for dynamic spinal support and alignment, preferably using minimally or less invasive techniques.

Historically, it has been common to fuse adjacent vertebrae that are placed in fixed relation by the installation therealong of bone screws or other bone anchors and cooperating longitudinal connecting members or other elongate members. Fusion results in the permanent immobilization of one or more of the intervertebral joints. Because the anchoring of bone screws, hooks and other types of anchors directly to a vertebra can result in significant forces being placed on the vertebra, and such forces may ultimately result in the loosening of the bone screw or other anchor from the vertebra, fusion allows for the growth and development of a bone counterpart to the longitudinal connecting member that can maintain the spine in the desired position even if the implants ultimately fail or are removed.

Because fusion has been a desired component of spinal stabilization procedures, longitudinal connecting members have been designed that are of a material, size and shape to largely resist flexion, extension, torsion, distraction and compression, and thus substantially immobilize the portion of the spine that is to be fused. Thus, longitudinal connecting members are typically uniform along an entire length thereof, and usually made from a single or integral piece of material having a uniform diameter or width of a size to provide substantially rigid support in all planes.

WO 2008/013892 describes a system for dynamic stabilization of a spine, comprising: at least two anchors and a rod dimensioned to be positioned between the two anchors, said rod having a tensioned cord situated entirely within an interior space of said rod which is defined by a bumper and a pair of couplers.

An alternative to fusion, which immobilizes at least a portion of the spine, and the use of more rigid longitudinal connecting members or other rigid structure has been a Asoft@ or Adynamic@ stabilization approach in which a flexible loop-, S-, C- or U-shaped member or a coil-like and/or a springlike member is utilized as an elastic longitudinal connecting member fixed between a pair of pedicle screws in an attempt to create, as much as possible, a normal loading pattern between the vertebrae in flexion, extension, distraction, compression, side bending and torsion. Another type of soft or dynamic system known in the art includes bone anchors connected by flexible cords or strands. Such a cord or strand may be threaded through cannulated spacers that are disposed between adjacent bone anchors when such a cord or strand is implanted, tensioned and attached to the bone anchors. The spacers typically span the distance between bone anchors, providing limits on the bending movement of the cord or strand and thus strengthening and supporting the overall system. However, such known systems have provided limited control with respect to torsional and shear forces.

### Summary of the Invention

A dynamic stabilization assembly according to the invention includes a flexible elongate inner core member, at least one spacer surrounding the member and at least a pair of opposed anti-torque sleeves and/or end caps disposed on either side of the spacer, each sleeve or end cap having an outer surface with at least one and up to a plurality of protrusions, grooves, ridges, notches or serrations and illustrated as radially extending ridges or teeth that form cooperating grooves or troughs therebetween. At least a pair of bone anchors cooperates with the elongate core member, with each bone anchor having at least one and up to a plurality of mating apertures, ridges or cooperating grooves formed on a side surface thereof to cooperatively receive and matingly frictionally engage with the protrusion or protrusions, ridges or grooves of a facing anti-torque/anti-shear sleeve, the engaging surfaces of the bone screws and the sleeves or end caps providing torsion and shear control of the assembly at each of the bone screws.

### Objects and Advantages of the Invention

An object of the invention is to provide dynamic medical implant stabilization assemblies having longitudinal connecting members that include a flexible portion that limits response to torsional and shear forces as well as allowing for controlled flexion, extension and compression of the assembly. A further object of the invention is to provide dynamic medical implant longitudinal connecting members that may be utilized with a variety of bone screws, hooks and other bone anchors. Additionally, it is an object of the invention to provide a lightweight, reduced volume, low profile assembly including at least two bone anchors and a longitudinal connecting member therebetween. Furthermore, it is an object of the invention to provide apparatus that are easy to use and especially adapted for the intended use thereof and wherein the apparatus are comparatively inexpensive to make and suitable for use.

It is a further object of the invention to provide a medical implant assembly having at least first and second bone anchors cooperating with a longitudinal connecting member, the improvement wherein the longitudinal connecting member comprises a tensioned flexible inner cord, the cord fixed to the at least first and second bone anchors; a compressible spacer having a through bore, the cord disposed in the bore and slidable with respect to the spacer along a central axis of the member; and a pair of opposed sleeves disposed on either side of the spacer, the spacer in sliding engagement with the first and second sleeves along the axis, each sleeve in fixed rotational relation with respect to the spacer, the cord extending through each sleeve, each sleeve having a side surface with at least one protrusion; and wherein each of the at least two bone anchors has a side surface with a protrusion receiving structure thereon, each sleeve frictionally engaging one of the bone screws at the at least one protrusion, substantially limiting rotation of the sleeves about the axis.

It is a further object of the invention to provide the improvement wherein at least one protrusion is a plurality of protrusions in the form of radially extending ridges and the protrusion receiving structure is a plurality of radially extending grooves.

It is a further object of the invention to provide the improvement wherein at least one of the bone screws is a closed monoaxial screw.

It is a further object of the invention to provide the improvement wherein at least one of the bone screws is an open monoaxial screw.

It is a further object of the invention to provide the improvement wherein each of the sleeves has at least one arm extending into the through bore of the spacer, the at least one arm frictionally engaging an inner surface of the spacer.

It is a further object of the invention to provide the improvement wherein the through bore is substantially rectangular in cross-section.

It is a further object of the invention to provide the improvement wherein the through bore is substantially square in cross-section.

It is a further object of the invention to provide the improvement wherein each of the sleeves has at least two arms extending into the through bore, each arm slidingly frictionally engaged with a planar surface forming the through-bore.

It is a further object of the invention to provide a medical implant assembly having at least first and second bone anchors holding an elongate cord under tension along a central axis and a spacer surrounding the cord, the spacer located between the first and second bone anchors, the improvement comprising a pair of opposed sleeves disposed on either side of the spacer, each sleeve disposed about the cord and having a portion thereof disposed between the cord and the spacer, fixing the sleeve to the spacer with respect to rotation about the axis, each sleeve having a side surface with a plurality of protrusions; and wherein each of the first and second bone anchors has a side surface with protrusion receiving structure thereon, each sleeve frictionally engaging one of the bone screws at the protrusions, substantially limiting sliding movement of the sleeves with respect to an engaged bone screw.

It is a further object of the invention to provide the improvement wherein the protrusions are radially extending ridges and the protrusion receiving structure is a plurality of radially extending grooves.

It is a further object of the invention to provide the improvement wherein at least one of the bone screws is a closed monoaxial screw.

It is a further object of the invention to provide the improvement wherein at least one of the bone screws is an open monoaxial screw.

It is a further object of the invention to provide the improvement wherein each of the sleeves has at least one arm extending into a through bore of the spacer, the at least one arm frictionally engaging an inner surface of the spacer.

It is a further object of the invention to provide the improvement wherein the through bore is substantially rectangular in cross-section.

It is a further object of the invention to provide the improvement wherein the through bore is substantially square in cross-section.

It is a further object of the invention to provide the improvement wherein each of the sleeves has at least two arms extending into the through bore, each arm slidingly frictionally engaged with a planar surface forming the through-bore.

It is a further object of the invention to provide a medical implant assembly having at least first and second bone anchors holding an elongate cord under tension along a central axis and a spacer surrounding the cord, the spacer located between the first and second bone anchors, the improvement comprising a pair of opposed sleeves disposed on either side of the spacer, each sleeve disposed about the cord and having a portion thereof disposed between the cord and the spacer, the portion fixing the sleeve to the spacer with respect to rotation about the axis, each sleeve having a side surface with a plurality of radially extending ridges; and wherein each of the first and second bone anchors has a side surface with a plurality of radially extending grooves thereon, each sleeve frictionally engaging one of the bone screws at the grooves and ridges.

It is a further object of the invention to provide the improvement wherein the spacer has a through bore of a polygonal cross-section for receiving the cord.

It is a further object of the invention to provide the improvement wherein the through bore is substantially square in cross-section.

It is a further object of the invention to provide the improvement wherein each of the sleeves has at least two arms extending into the through bore, each arm slidingly frictionally engaged with a planar surface forming the through-bore.

It is a further object of the invention to provide a medical implant assembly having at least first and second bone anchors cooperating with a longitudinal connecting member, the improvement wherein the longitudinal connecting member comprises a flexible inner core, the core cooperating with the bone anchors and being sized and shaped to provide fixation between the bone anchors; a spacer having first and second ends and a through-bore extending from the first end to the second end, the core being located in the bore and slidable with respect to the spacer along a central axis of the longitudinal connecting member; and at least one sleeve disposed on an end of the spacer and in sliding engagement with the spacer along the central axis, the sleeve having a first end with a portion projecting into the spacer, the core extending through said sleeve, said sleeve having a second end with an external surface portion sized and shaped for frictional engagement with a bone anchor; and wherein engagement of the sleeve second end with the bone anchor and engagement of the sleeve first end portion with the spacer resists sliding shear movement between the spacer and the bone anchor.

It is a further object of the invention to provide the improvement wherein the sleeve second end includes a plurality of protrusions in the form of radially extending ridges and bone anchor includes a protrusion-receiving structure having a plurality of radially extending grooves.

It is a further object of the invention to provide the improvement wherein at least one of the bone screws is a closed monoaxial screw.

It is a further object of the invention to provide the improvement wherein at least one of the bone screws is an open monoaxial screw.

It is a further object of the invention to provide the improvement wherein the sleeve has at least one projection extending into the through bore of the spacer, the at least one projection frictionally engaging an inner surface of the spacer.

It is a further object of the invention to provide the improvement wherein the through bore is substantially rectangular in cross-section.

It is a further object of the invention to provide the improvement wherein the sleeve has at least two arms extending into the through bore, the arm being slidingly frictionally engaged with a planar surface forming the through-bore.

It is a further object of the invention to provide the improvement wherein the through bore is substantially square in cross-section.

It is a further object of the invention to provide a medical implant assembly having at least one pair of bone anchors, said assembly comprising a longitudinal core member cooperating with said pair of bone anchors to provide fixation therebetween and; at least one sleeve member with a first portion having a bone anchor frictional engagement surface; said sleeve member having a second portion with a projection extending away from said first portion engagement surface and a bore along the entire length of said sleeve member and surrounding said core member, said second portion projection extending into a spacer member, said spacer member having a bore along the entire length thereof surrounding said core member and positioned between said pair of bone anchors; and wherein said sleeve member first and second portions cooperate with said spacer member and at least one bone anchor to resist sliding shear movement between said spacer member and said bone anchor when said sleeve member first portion engagement surface is in frictional engagement with said bone anchor and said sleeve member second portion projection is engaged within said spacer member.

It is a further object of the invention to provide the improvement wherein said sleeve member first portion includes a flange having a cross-section wider than a cross-section of said sleeve member second portion projection.

It is a further object of the invention to provide the improvement wherein said sleeve member flange has a side surface with at least one protrusion.

It is a further object of the invention to provide the improvement wherein said sleeve member second portion projection is in fixed relation within said spacer.

It is a further object of the invention to provide the improvement wherein said core member is a cord.

It is a further object of the invention to provide the improvement wherein said core member is fixed to said bone anchors.

It is a further object of the invention to provide the improvement wherein said core member is tensioned.

It is a further object of the invention to provide a medical implant assembly having at least first and second bone anchors cooperating with a longitudinal connecting member, the assembly wherein the longitudinal connecting member has a central axis and comprises a flexible inner cord extending between at least the first and second bone anchors; a spacer having a through bore, the cord disposed in the bore and slidable with respect to the spacer along the member central axis; and at least one sleeve disposed on either side of the spacer such that each sleeve is in sliding engagement with the spacer and in overlapping relation with respect to the spacer, the cord extending through the sleeve; and wherein each sleeve has a protrusion extending into the spacer and each sleeve cooperates with at least one bone anchor to resist sliding shear movement between the spacer and the anchor.

It is a further object of the invention to provide a medical implant assembly having at least first and second bone anchors cooperating with a longitudinal connecting member, the assembly wherein the longitudinal connecting member has a central axis and comprises a flexible inner cord extending between at least the first and second bone anchors; a spacer having a through bore, the cord disposed in the bore and slidable with respect to the spacer along the member central axis; and at least one sleeve disposed on either side of the spacer such that each sleeve is in sliding engagement with the spacer and in overlapping relation with respect to the spacer, the cord extending through the sleeve; and wherein each sleeve member is positioned between a spacer and a bone anchor, the member being in overlapping relation with respect to the spacer, and wherein the member cooperates with the cord, the spacer and the anchor to resist sliding shear movement between the spacer and the bone anchor.

Other objects and advantages of this invention will become apparent from the following description taken in conjunction with the accompanying drawings wherein are set forth, by way of illustration and example, certain embodiments of this invention.

The drawings constitute a part of this specification and include exemplary embodiments of the present invention and illustrate various objects and features thereof.

### Brief Description of the Drawings

Fig. 1 is an enlarged perspective view of a dynamic stabilization assembly of the invention having an inner flexible core, an outer spacer, a pair of anti-torque sleeves and shown with one closed anti-torque bone screw and one open anti-torque bone screw of the invention.
Fig. 2 is a partial cross-sectional view taken along the line 2-2 of Fig. 1.
Fig. 3 is an enlarged side elevational view of the assembly of Fig. 1 with the spacer shown in phantom.
Fig. 4 is an enlarged perspective view of one of the anti-torque sleeves of Fig. 1.
Fig. 5 is an enlarged side elevational view of the anti-torque sleeve of Fig. 4.
Fig. 6 is an enlarged rear elevational view of the anti-torque sleeve of Fig. 4.
Fig. 7 is an enlarged front elevational view of the anti-torque sleeve of Fig. 4.
Fig. 8 is an enlarged, exploded and partial side elevational view, showing the anti-torque sleeve of Fig. 5 and the inner flexible core of Fig. 1 in a first stage of assembly.
Fig. 9 is an enlarged and partial side elevational view with portions broken away to show the detail thereof, showing the anti-torque sleeve of Fig. 5, the inner flexible core and the spacer of Fig. 1 in a further stage of assembly.
Fig. 10 is an enlarged and partial side elevational view, showing the sleeve, core and spacer of Fig. 9 in a further stage of assembly.
Fig. 11 is an enlarged and partially exploded side elevational view, showing a further stage of assembly between the assembled core, spacer and sleeves of Fig. 10 with the bone screws of Fig. 1.

### Detailed Description of the Invention

As required, detailed embodiments of the present invention are disclosed herein; however, it is to be understood that the disclosed embodiments are merely exemplary of the invention, which may be embodied in various forms. Therefore, specific structural and functional details disclosed herein are not to be interpreted as limiting, but merely as a basis for the claims and as a representative basis for teaching one skilled in the art to variously employ the present invention in virtually any appropriately detailed structure. It is also noted that any reference to the words top, bottom, up and down, and the like, in this application refers to the alignment shown in the various drawings, as well as the normal connotations applied to such devices, and is not intended to restrict positioning of the connecting member assemblies of the application and cooperating bone anchors in actual use.

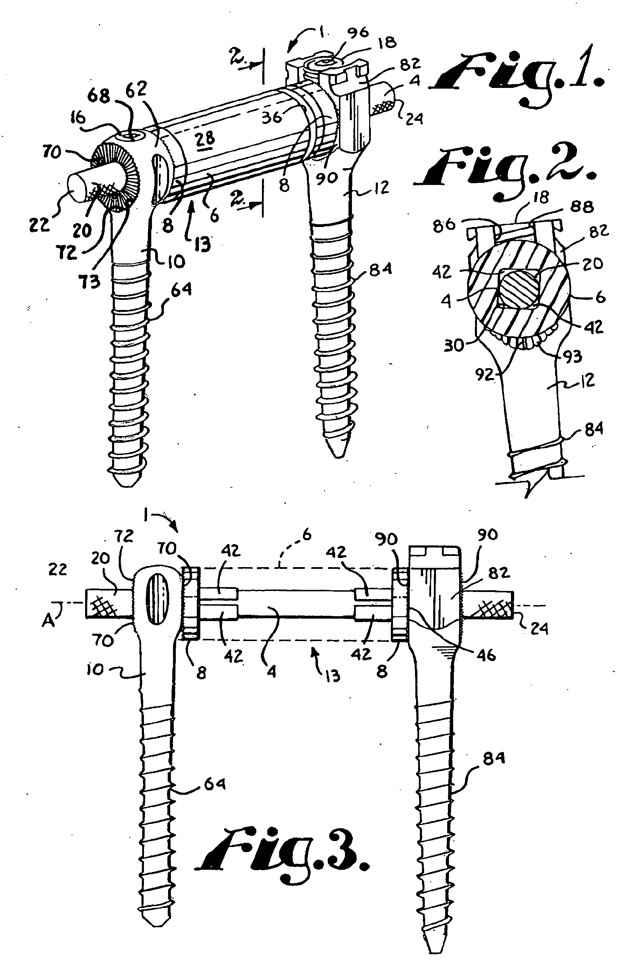

With reference to Figs. 1-11, the reference numeral 1 generally designates a non-fusion dynamic stabilization assembly of the invention. The illustrated assembly 1 includes the following components: an elongate flexible core in the form of a cord 4; at least one cannulated spacer 6; a pair of anti-torque/anti-shear sleeves or end caps 8; a closed anti-torque bone screw 10; and an open anti-torque bone screw 12. The elongate inner cord core 4 is slidingly receivable within the spacer 6, sleeves 8 to form a connecting member, generally 13, and is eventually tensioned and fixed to each of the bone screws 10 and 12. Each sleeve 8 engages the spacer 6 on one side thereof and a bone screw 10 or 12 on an opposed side thereof. As will be described in greater detail below, when fully assembled and all the components are fixed in position as shown in Figs. 1-3, for example, the core 4 is in tension and the spacer 6 may be in compression or in a neutral state, the cord 4 and spacer 6 combination providing for protected spinal movement in spinal flexion and extension, for example, with the cooperating sleeves 8 and screw 10 and 12 engaging to control torsion or twisting movement and limit rotation of the individual components 6 and 8 of the assembly 1 with respect to one another and with respect to the bone screws 10 and 12 generally along and about an axis A of the assembly 1.

As illustrated, for example, in Figs. 1-3, the dynamic connecting member assembly 1 includes at least two bone anchors and is illustrated with one fixed or monoaxial closed screw 10 cooperating with a set screw 16 and one fixed or monoaxial, open screw 12 cooperating with a closure top 18, the assembly 1 being captured and fixed in place at portions of the cord 4 located on either side of the spacer 6 and cooperating sleeves 8. Although the screws 10 and 12 are illustrated, it is noted that the assembly 1 may be used with two or more screws 10 or two or more screws 12 or any combination of the screws 10 and 12. Also, although both the screws 10 and 12 are fixed or monoaxial screws, a variety of bone screws and other bone anchors may be modified to include surfaces for cooperation with the sleeves 8, including hinged bone screws, polyaxial bone screws, and bone hooks and the like, with or without compression inserts, that may in turn cooperate with a variety of closure structures having threads, flanges, or other structure for fixing the closure structure to the bone anchor, and may include other features, for example, external or internal drives, break-off tops and inner set screws. The bone anchors, closure structures and the connecting member 13 are then operably incorporated in an overall spinal implant system for correcting degenerative conditions, deformities, injuries, or defects to the spinal column of a patient.

The connecting member 13 is elongate, with the inner core 4 being any flexible elongate material including, but not limited to cords, threads, strings, bands, cables or fibers that may be single or multiple strands, including twisted, braided or plaited materials. The illustrated cord 4 has a substantially uniform body 20 of substantially circular cross-section, a first end 22 and an opposed second end 24, the cord 4 being cut to length as required by the surgeon. Initially, the cord 4 is typically of a length longer than shown in the drawings to allow for gripping of the cord 4 during assembly with the other components of the connecting member 13 and also for tensioning and attachment to the bone screws of the assembly 1 as will be described in greater detail below. The cord 4 may be made from a variety of materials, including polyester or other plastic fibers, strands or threads, such as polyethylene-terephthalate. The cord 4 may be placed under axial tension prior to final installation between the bone screws 10 and 12, for example by being tensioned along the axis A for a selected time to lengthen and otherwise deform the cord 4 during a primary creep stage. After the cord 4 reaches a secondary or steady-state creep, further tension may then be placed on the cord 4 in preparation for fixing to the bone screws 10 and 12 as will be described in greater detail below. It is noted that the cord 4 typically does not illustrate elastic properties, such as any significant additional axial distraction, after the assembly 1 is operatively assembled within a human body.

With particular reference to Figs. 1-3 and 11, the spacer 6 is sized and shaped to be slidingly received over the cord 4 and portions of the sleeves 8 and may be made from a variety of elastic materials, including, but not limited to natural or synthetic elastomers such as polyisoprene (natural rubber), and synthetic polymers, copolymers, and thermoplastic elastomers, for example, polyurethane elastomers such as polycarbonate-urethane elastomers. In order to have low or no wear debris, the spacer 6 inner and side surfaces may be coated with an ultra thin, ultra hard, ultra slick and ultra smooth coating, such as may be obtained from ion bonding techniques and/or other gas or chemical treatments. The illustrated spacer 6 has an external substantially cylindrical outer surface 28 and an internal surface 30 defining a through bore. The inner surface 30 is substantially rectangular or square in a cross-section taken perpendicular to the axis A and as shown in Fig. 2. The surface 30 is sized and shaped to closely cooperate and fit about extended arm portions of the sleeves 8 as will be described in greater detail below. The spacer 6 includes opposed substantially planar and annular end surfaces 32 and 34 that are sized and shaped to abut against planar surfaces of the sleeves 8. When initially assembled with the other components of the connecting member 13, the surfaces 32 and 34 are substantially perpendicular to the axis A. It is foreseen that in some embodiments, the spacer 6 may be of smaller or larger outer circular cross section, or of a square, rectangular or other inner or outer cross-section including other curved or polygonal shapes. The spacer 6 includes a compression groove 36. Spacers according to the invention may include one, none or any number of grooves that allow for some additional compression of the spacer 6 when pressed upon in an axial direction between the bone anchors 10 and 12. The illustrated groove 36 is substantially uniform and circular in cross-section, being formed in the external surface 28 and extending radially toward the internal surface 30. The size of the internal surface 30 allows for some axially directed sliding movement of the spacer 6 with respect to the cord 4.

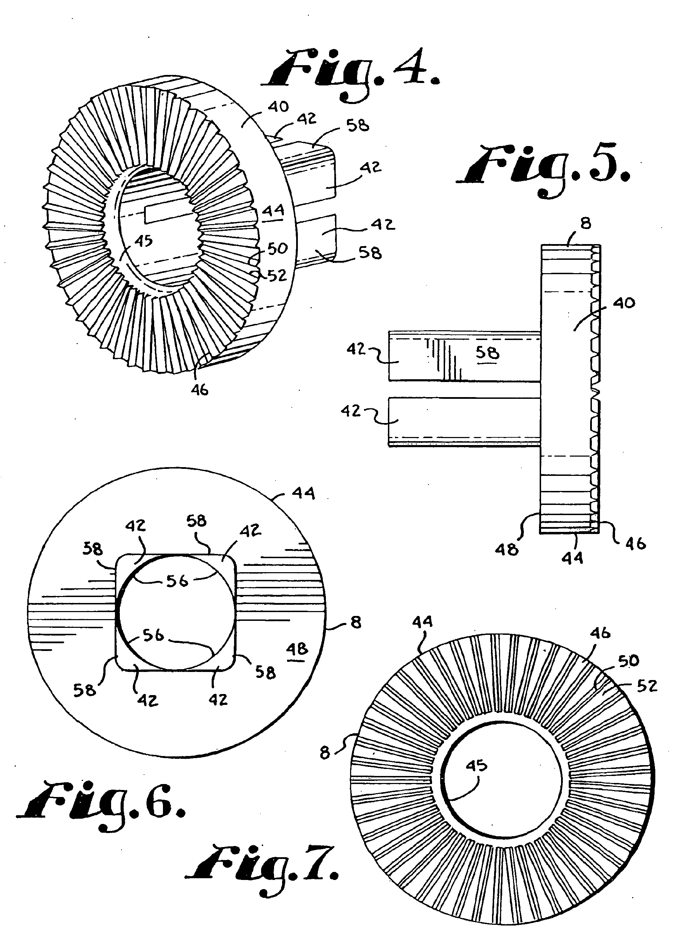

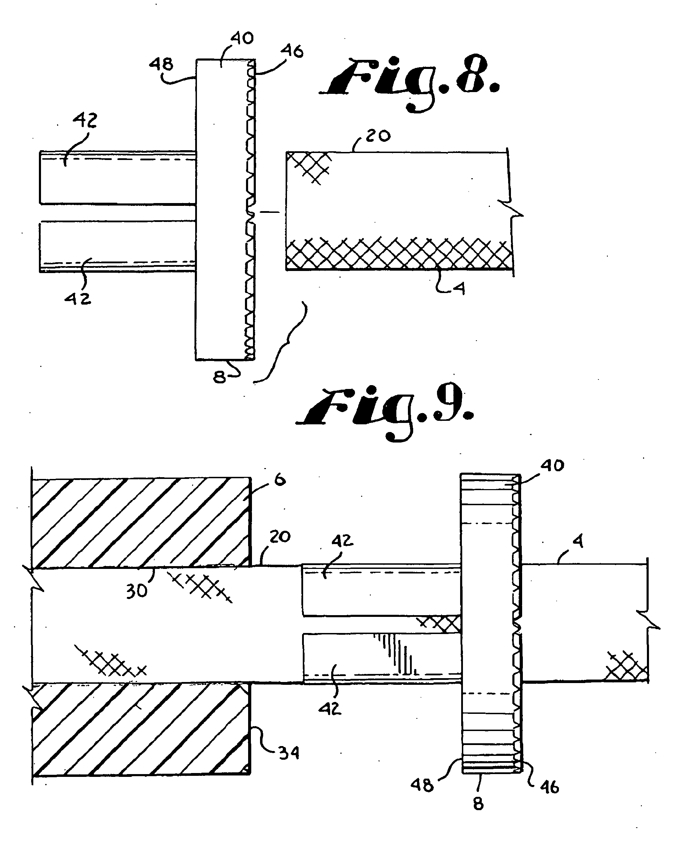

With particular reference to Figs. 4-7, each sleeve 8 includes a substantially cylindrical body 40 having integral extension arms 42. The body 40 is annular and includes an outer cylindrical surface 44, an inner cylindrical surface 45, an end surface 46 and an opposed end surface 48, the arms 42 extending from the surface 48. The inner cylindrical surface 45 forms a bore sized and shaped for closely receiving the cord 4 therethrough as shown, for example, in Figs. 8 and 9. The end surfaces 46 and 48 are substantially parallel to one another. The end surface 46 includes radially extending ridges 50 forming grooves 52 therebetween, the ridges 50 and grooves 52 cooperating with and frictionally engaging surfaces of the bone screws 10 and 12 as will be discussed in greater detail below.

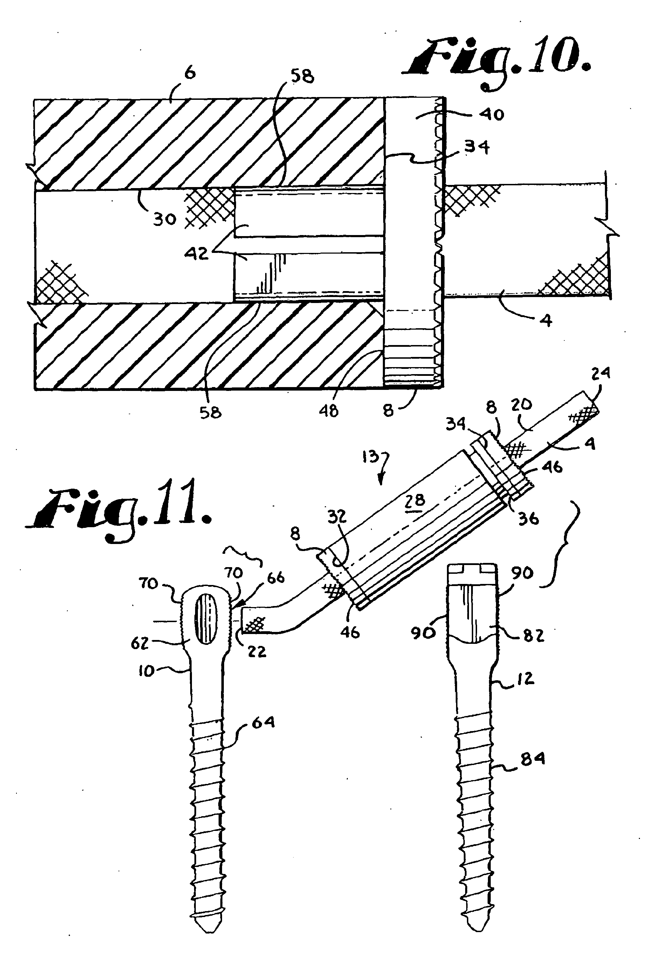

In the illustrated embodiment, there are four extension arms 42 integral with the sleeve body 40. It is noted, however, that in other embodiments fewer arms may be used, for example, two opposed arms. The arms 42 are substantially identical, each having an inner curved surface 56 and an outer surface 58 formed by a pair of adjoining planar surfaces disposed perpendicular to one another and sized and shaped to be closely received in corners of the internal surface 30 of the spacer 6. Thus, when received within the spacer 6, the extension arms 42 fit substantially squarely within the surface 30 and any rotation of the sleeve 8 with respect to the spacer 6 is prohibited. Also, when engaged with the spacer 6 as shown, for example, in Figs. 2 and 10, the inner curved surfaces 56 substantially form a discontinuous inner cylindrical surface for closely receiving the cord 4.

The sleeves 8 may be made from metal, metal alloys or other suitable materials, including plastic polymers such as polyetheretherketone (PEEK), ultra-high-molecular weight-polyethylene (UHMWP), polyurethanes and composites, including composites containing carbon fiber. It is noted that the sleeves 8 are preferably made from a different material than the bone screws 10 and 12, for example, titanium bone screws advantageously cooperate with sleeves 8 made from PEEK. In order to have low or no wear debris, the sleeve 8 end surfaces 46 and 48 and/or engaging, cooperating bone screw 10 and 12 surfaces may be coated with an ultra thin, ultra hard, ultra slick and ultra smooth coating, such as may be obtained from ion bonding techniques and/or other gas or chemical treatments.

The bone screw 10 with cooperating set screw 16 is a monoaxial screw having an upper cord receiving portion 62 integral with a threaded bone attachment portion or shank 64. The portion 62 further includes a first through bore 66 for closely receiving the cord 4 therethrough and a second threaded bore 68 for receiving and mating with the set screw 16, the bore 68 disposed in a direction substantially perpendicular to the first through bore 66 so that the set screw 16 engages the cord 4 and fixes the cord 4 to the screw 10. The upper, receiving portion 62 further includes opposed, substantially parallel side surfaces 70. However, it is foreseen that according to the invention, other embodiments of the invention may include side surfaces 70 that angle away or towards one another for lordosing or kyphosing controlling embodiments as previously described in applicant's application US 2006/0111715. Each of the surfaces 70 further include radially extending ridges 72 forming grooves 73 therebetween, the ridges 72 being sized and shaped for engaging the grooves 52 of a cooperating sleeve 8 and the grooves 73 for receiving and engaging the radially extending ridges 50 of such sleeve 8.

The bone screw 12 with cooperating closure top 18 16 is an open, fixed, monoaxial screw having an upper cord receiving portion 82 integral with a threaded bone attachment portion or shank 84. The portion 82 further includes a substantially U-shaped channel 86 for closely receiving the cord 4 therethrough, the channel 86 further having an upper closure top receiving portion with a helically wound guide and advancement structure 88 thereon for receiving and mating with the closure top 18, the closure 18 engaging the cord 4 and fixing the cord 4 to the screw 12. The upper, receiving portion 82 further includes opposed, substantially parallel side surfaces 90. However, it is foreseen that according to the invention, other embodiments of the invention may include side surfaces 90 that angle away or towards one another for lordosing or kyphosing controlling embodiments as previously described in applicant's application US 2006/0111715. Each of the surfaces 90 further include radially extending ridges 92 forming grooves 93 therebetween, the ridges 92 being sized and shaped for engaging the grooves 52 of a cooperating sleeve 8 and the grooves 93 for receiving and engaging the radially extending ridges 50 of such sleeve 8.

It is noted that the sleeve 8 surfaces 46 and cooperating bone screw 10 surfaces 70 and bone screw 12 surfaces 90 may have other types of engaging surfaces, such as cooperating protrusions and notches and other surface geometries that frictionally engage and thus limit or prohibit rotation of the sleeve 8 about the axis A when the sleeve 8 is engaged with the bone screw 10 or the bone screw 12.

To provide a biologically active interface with the bone, the threaded shanks 64 and 84 of the respective bone screws 10 and 12 may be coated, perforated, made porous or otherwise treated. The treatment may include, but is not limited to a plasma spray coating or other type of coating of a metal or, for example, a calcium phosphate; or a roughening, perforation or indentation in the shank surface, such as by sputtering, sand blasting or acid etching, that allows for bony ingrowth or ongrowth. Certain metal coatings act as a scaffold for bone ingrowth. Bio-ceramic calcium phosphate coatings include, but are not limited to: alpha-tri-calcium phosphate and beta-tri-calcium phosphate (Ca₃(PO₄)₂, tetra-calcium phosphate (Ca₄P₂O₉), amorphous calcium phosphate and hydroxyapatite (Ca₁₀(PO₄)₆(OH)₂). Coating with hydroxyapatite, for example, is desirable as hydroxyapatite is chemically similar to bone with respect to mineral content and has been identified as being bioactive and thus not only supportive of bone ingrowth, but actively taking part in bone bonding.

With reference to Figs. 1 and 2, the closure structure 18 can be any of a variety of different types of closure structures for use in conjunction with the present invention with suitable mating structure on the interior surface 88 of the receiver 82 of the open bone screw 12. The illustrated closure structure 18 is rotatable between the spaced arms forming the receiver 82. The illustrated structure 18 is substantially cylindrical and includes an outer helically wound guide and advancement structure in the form of a flange form that operably joins with the guide and advancement structure 88. A driving tool (not shown) sized and shaped for engagement with an internal drive feature 96 is used for both rotatable engagement and, if needed, disengagement of the closure 18 from the receiver 82. The internal drive feature 96 may take a variety of forms and may include, but is not limited to, a hex shape, TORX or other features or apertures, such as slotted, tri-wing, spanner, two or more apertures of various shapes, and the like.

In use, the two bone screws 10 and 12 are implanted into vertebrae for use with the dynamic connecting member 13. Each vertebra may be pre-drilled to minimize stressing the bone. Furthermore, if a cannulated bone screw shank is utilized, each vertebra will have a guide wire or pin (not shown) inserted therein that is shaped for the bone screw cannula of the bone screw shank 64 of 84 and provides a guide for the placement and angle of the shank 64 or 84 with respect to the cooperating vertebra. A further tap hole may be made and the shank 64 or 84 is then driven into the vertebra by rotation of a driving tool (not shown) that engages a driving feature on or near the top portion 62 or 82 of the respective screw 10 or 12. It is foreseen that the screws 10 and 12 and the dynamic connector 13 can be inserted in a percutaneous or minimally invasive surgical manner.

With particular reference to Figs. 8-11, the dynamic connector 13 may be assembled by inserting the cord 4 into one of the sleeves 8 as shown in Figs. 8 and 9, the cord being threaded through the inner cylindrical surface 45 and the inner surfaces 56 of the arms 42 of the sleeve 8, followed by insertion of the cord 4 into the bore formed by the spacer internal surface 30. The spacer 6 is slid along the cord 4 until the spacer end surface 34 abuts against the sleeve 8 end surface 48 with the extension arms 42 of the sleeve 8 being disposed within the lumen of the spacer 6 formed by the internal surface 30 having a substantially square cross-section. At this time the sleeve 8 and the spacer 6 are axially slidable with respect to one another, for example with respect to the axis A and as illustrated in Fig. 2, but fixed with respect to rotation about the axis A.

Then, a second sleeve 8 is threaded onto the cord 4 with the extension arms 42 thereof facing the spacer 6. The second sleeve 8 is slid along the cord 4 until the end surface 48 thereof abuts against the end surface 32 of the spacer 6 with the extension arms 42 of the second sleeve 8 being in sliding engagement with the internal surface 30. As with the first sleeve, the second sleeve 8 is now axially slidable with respect to the spacer 6 but rotation of the sleeve 8 with respect to the spacer 6 is prohibited. The dynamic connector 13 is now assembled and ready for placement between two bone screws 10 and 12 as illustrated in Fig. 11 with serrated sleeve surfaces 46 directed outwardly.

Also as indicated in Fig. 11, the cord 4 is first received into the bore 66 of the closed bone screw 10 and the set screw 16 is rotated and driven into the bore 68 until the set screw 16 frictionally engages the cord 4 and fixes the cord 4 to the screw 10. The spacer 6 and two sleeves 8 are then positioned between the bone screw 10 and the bone screw 12 with the sleeve end surfaces 46 engaging the surface 70 of the bone screw 10 at one end of the assembly 13 and with the surface 90 of the bone screw 12 at the other end of the assembly as also shown in Figs. 1 and 3. The cord 4 is tensioned and the spacer 6 may be compressed as described and illustrated in US 2006/0111715. The closure top 18 is then inserted into the receiver 82 of the screw 12, rotated and tightened to secure the tensioned cord within the receiver 82.

The resulting connecting member assembly 1 is thus dynamically loaded with the cord 4 in tension and the radially extending ridges and grooves of the sleeve end surfaces 46 in frictional engagement with both the ridges and grooves of the bone screw surface 70 and the ridges and grooves of the bone screw surface 90, preventing or substantially limiting rotation of the spacer 6 and engaged sleeves 8 about the axis A and any other sliding movement between the spacer 6 and the sleeves 8. The assembly 1 is thus substantially dynamically loaded and oriented relative to the cooperating vertebra, providing relief (e.g., shock absorption), controlling torsional and shear forces and providing protected movement with respect to flexion, extension, distraction and compressive forces placed on the assembly 1.

If removal of the dynamic connector 13 from the bone screws 10 and/or 12 is necessary, or if it is desired to release the assembly 13 at a particular location, disassembly is accomplished by using the driving tool (not shown) with a driving formation cooperating with the set screw 16 or the closure structure 18 to rotate and remove the respective set screw or closure structure from the respective bone screw 10 and/or 12. Disassembly is then accomplished in reverse order to the procedure described previously herein for assembly.

It is to be understood that while certain forms of the present invention have been illustrated and described herein, it is not to be limited to the specific forms or arrangement of parts described and shown.

## Claims

1. A medical implant assembly (1) having at least first and second bone anchors (10, 12) cooperating with a longitudinal connecting member (13) along the axis (A), wherein the longitudinal connecting member (13) includes a tensioned deformable inner cord (4) securable to the bone anchors (10, 12) and a spacer (6) having a through bore with the cord (4) disposed therethrough, the spacer (6) being slidable with respect to the cord (4) along a central axis (A) of the longitudinal connecting member (13), the medical implant assembly (1) **characterized by** having:
a) a sleeve (8) disposed on an end (32, 34) of the spacer (6) and about the cord (4), the sleeve (8) having a portion thereof disposed between the cord (4) and the spacer (6), the sleeve (8) having a first end surface (48) that abuts against the end (32, 34) of the spacer (6), so as to resist sliding shear, the sleeve (8) having a second end surface (46) with at least one radially extending ridge (50) ; wherein
b) the radially extending ridge (50) is sized and shaped to engage a groove (73) being located on one of the bone anchors (10, 12) to both limit rotation of the spacer (6) and the sleeve(8) about the axis A and sliding movement between the spacer(6) and sleeve(8), so as to control torsional and shear forces.

2. The assembly (1) of claim 1 characterised wherein:
each of the bone anchors (10, 12) has a side surface (70, 90) with a protrusion receiving structure thereon, each sleeve (8) frictionally engaging one of the bone anchors (10, 12) at the protrusion, thereby substantially limiting rotation of the sleeves (8) about the axis (A).

3. The assembly (1) of claim 1 characterized wherein:
the at least one protrusion is a plurality of protrusions in the form of radially extending ridges (50) and the protrusion receiving structure is a plurality of radially extending grooves (73, 93).

4. The assembly (1) of claim 1 characterized wherein:
the sleeve (8) has at least one arm (42) extending into the spacer through bore, the at least one arm (42) frictionally engaging an inner surface (30) of the spacer.

5. The assembly (1) of claim 4 characterized wherein:
the spacer through bore is substantially rectangular in cross-section.

6. The assembly (1) of claim 5 characterized wherein:
the sleeve (8) has at least two arms (42) extending into the spacer through bore, each arm (42) being slidingly frictionally engaged with a planar surface (30) forming the spacer through bore.

## Patentansprüche

1. Medizinische Implantatanordnung (1), die zumindest erste und zweite Knochenanker (10, 12) besitzt, die mit einem Längsverbindungsteil (13) entlang der Achse (A) zusammenwirken, wobei das Längsverbindungsteil (13) ein gespanntes, verformbares inneres Kabel (4) umfasst, das an die Knochenanker (10, 12) befestigbar ist, und einen Abstandhalter (6), der eine durchgehende Bohrung, wobei durch diese hindurch das Kabel (4) angeordnet ist, besitzt, wobei der Abstandhalter (6) relativ zu dem Kabel (4) entlang einer mittleren Achse (A) des Längsverbindungsteils (13) verschieblich ist, wobei die medizinische Implantatanordnung (1) **dadurch gekennzeichnet ist, dass** sie folgendes aufweist:
a) eine Hülse 8, die an einem Ende (32, 34) des Abstandhalters (6) und um das Kabel (4) angeordnet ist, wobei die Hülse (8) einen Tiel hiervon zwischen dem Kabel (4) und dem Abstandhalter (6) angeordnet hat, wobei die Hülse eine erste Endoberfläche (48) besitzt, die am Ende (32, 34) des Abstandhalters (6) anliegt, so dass sie Verschiebungs-Schubspannung widersteht, wobei die Hülse (8) eine zweite Endoberfläche (46) mit mindestens einem sich radial erstreckenden Grat (50) besitzt,
b) wobei der sich radial erstreckenden Grat (50) so bemessen und geformt ist, dass er in eine Nut (73) eingreift, die sich auf einem der Knochenanker (10, 12) befindet, um sowohl Drehung des Abstandhalters (6) und der Hülse (8) um die Achse (A) zu begrenzen, als auch eine verschiebende Bewegung des Abstandhalters (6) und der Hülse (8), um so Torsions- und Scherkräfte zu steuern.

2. Anordnung (1) gemäß Anspruch 1, **dadurch gekennzeichnet, dass** jeder der Knochenanker (10, 12) eine Seitenfläche (70, 90) mit einer überstandaufnehmenden Struktur darauf besitzt, wobei jede Hülse (8) reibend mit einem der Knochenanker (10, 12) an dem Überstand in Kontakt steht und dadurch die Drehung der Hülsen (8) um die Achse (A) wesentlich begrenzt.

3. Anordnung (1) gemäß Anspruch 1, **dadurch gekennzeichnet, dass** der mindestens eine Überstand als mehrere Überstände in Form von sich radial erstreckenden Graten (50) ausgeführt ist, und die überstandaufnehmende Struktur als mehrere sich radial erstreckende Nuten (73, 93) ausgeführt ist.

4. Anordnung (1) gemäß Anspruch 1, **dadurch gekennzeichnet, dass** die Hülse (8) zumindest einen Arm (42) besitzt, der sich in die durchgehende Bohrung des Abstandhalters erstreckt, wobei der mindestens eine Arm (42) reibend mit einer inneren Oberfläche (30) des Abstandhalters in Kontakt steht.

5. Anordnung (1) gemäß Anspruch 4, **dadurch gekennzeichnet, dass** die durchgehende Bohrung des Abstandhalters im Querschnitt im wesentlichen rechteckig ist.

6. Anordnung (1) gemäß Anspruch 5, **dadurch gekennzeichnet, dass** die Hülse (8) zumindest zwei Arme (42) besitzt, die sich in die durchgehende Bohrung des Abstandhalters erstrecken, wobei jeder Arm (42) verschieblich reibend mit einer ebenen Oberfläche (30) in Kontakt steht, die die durchgehende Bohrung des Abstandhalters bildet.

## Revendications

1. Ensemble d'implant médical (1) comprenant au moins un premier et un deuxième ancrages d'os (10, 12) qui coopèrent avec un élément de connexion longitudinal (13) le long de l'axe (A), dans lequel l'élément de connexion longitudinal (13) comprend un cordon intérieur déformable tendu (4) qui peut être fixé aux ancrage d'os (10, 12), et un écarteur (6) présentant un alésage traversant au travers duquel le cordon (4) est disposé, l'écarteur (6) pouvant coulisser par rapport au cordon (4) le long d'un axe central (A) de l'élément de connexion longitudinal (13), l'ensemble d'implant médical (1) étant **caractérisé en ce qu'**il comprend:
a) un manchon (8) qui est disposé sur une extrémité (32, 34) de l'écarteur (6) et autour du cordon (4), le manchon (8) comprenant une partie qui est disposée entre le cordon (4) et l'écarteur (6), le manchon (8) présentant une première surface d'extrémité (48) qui bute contre l'extrémité (32, 34) de l'écarteur (6), de manière à résister au cisaillement glissant, le manchon (8) présentant une deuxième surface d'extrémité (46) comportant au moins une nervure s'étendant radialement (50), et dans lequel:
b) la nervure s'étendant radialement (50) est dimensionnée et configurée de manière à engager une rainure (73) située sur l'un des ancrages d'os (10, 12) afin de limiter à la fois la rotation de l'écarteur (6) et du manchon (8) autour de l'axe (A) et le mouvement coulissant entre l'écarteur (6) et le manchon (8), de manière à régler les forces de torsion et de cisaillement.

2. Ensemble (1) selon la revendication 1, **caractérisé en ce que** chacun des ancrages d'os (10, 12) présente une surface latérale (70, 90) présentant une structure de réception de saillie, chaque manchon (8) engageant par frottement un des ancrages d'os (10, 12) au niveau de la saillie, limitant de ce fait sensiblement la rotation des manchons (8) autour de l'axe (A).

3. Ensemble (1) selon la revendication 1, **caractérisé en ce que** ladite au moins une saillie est une pluralité de saillies sous la forme de nervures s'étendant radialement (50), et la structure de réception de saillie est une pluralité de rainures s'étendant radialement (73, 93).

4. Ensemble (1) selon la revendication 1, **caractérisé en ce que** le manchon (8) comprend au moins un bras (42) qui s'étend dans l'alésage traversant de l'écarteur, ledit au moins un bras (42) engageant avec frottement une surface intérieure (30) de l'écarteur.

5. Ensemble (1) selon la revendication 4, **caractérisé en ce que** la section transversale de l'alésage traversant de l'écarteur est sensiblement rectangulaire.

6. Ensemble (1) selon la revendication 5, **caractérisé en ce que** le manchon (8) comprend au moins deux bras (42) qui s'étendent dans l'alésage traversant de l'écarteur, chaque bras (42) étant engagé avec frottement de façon coulissante avec une surface plane (30) qui forme l'alésage traversant de l'écarteur.
